# EUROPEAN PATENT APPLICATION

(11) **EP 2 058 672 A1**
(43) Date of publication of application: **13.05.2009**
(21) Application number: 08019231.3
(22) Date of filing: 04.11.2008
(51) Int. Cl.: G01S 7/52, G06T 15/00

(54) **Ultrasound imaging system including a graphic processing unit**

(30) Priority: 09.11.2007 KR 20070114551
(71) Applicant: MEDISON CO., LTD., Kangwon-do 250-870 (KR)
(72) Inventor: Lee, Suk, Jin, Gangnam-gu Seoul 135-280 (KR); Kim, Hyoung Jin, Gangnam-gu Seoul 135-280 (KR); Lee, Jae, Keun, Gangnam-gu Seoul 135-280 (KR)
(74) Representative: Schmid, Wolfgang

(57) **Abstract**

The present invention relates to an ultrasound imaging system. The ultrasound imaging system includes an ultrasound diagnostic unit and an image processing unit. The ultrasound diagnostic unit transmits ultrasound signals to a target object and forms receive data based on ultrasound echo signals reflected from the target object. The image processing unit forms an ultrasound image based on the receive data. The image processing unit includes a graphic processing unit configured to perform at least one of functions including processing the receive data to form image data, performing scan conversion upon the image data to form scan-converted data suitable for display, and rendering and filtering the scan-converted data to form pixel data.

## Description

The present application claims priority from Korean Patent Application No. 10-2007-0114551 filed on November 09, 2007, the entire subject matters of which are incorporated herein by reference.

### BACKGROUND OF THE INVENTION

### [Technical Field]

The present invention generally relates to an ultrasound imaging system, and more particularly to an ultrasound imaging system including a graphic processing unit (GPU).

### [Background Art]

An ultrasound imaging system has become an important and popular diagnostic tool due to its non-invasive and non-destructive nature. The ultrasound imaging system may form an ultrasound image based on reflection, scattering and absorption of ultrasound signals when the ultrasound signals are propagated into tissues of a target object.

The ultrasound imaging system may include an ultrasound diagnostic unit and an imaging processing unit. The ultrasound diagnostic unit may transmit ultrasound signals to a target object and form 8-bit receive data based on echo signals. The imaging processing unit may form an ultrasound image based on the receive data. The image processing unit may include a digital signal processing unit (DSP), a digital scan converter (DSC) and a central processing unit (CPU). The DSP may be operable to process the receive data to form 8-bit raw data for forming a brightness (B) mode image, a color (C) mode image or a Doppler (D) mode image. The DSC may be operable to scan-convert the raw data to thereby output scan-converted data suitable for a display format. The CPU may be operable to control operations of the DSP, DSC and a display unit. Also, the CPU may be further operable to perform filtering and rendering upon the scan-converted data to thereby form pixel data for image modes.

The rendering and formation of the pixel data performed in the CPU may require a large amount of data operations so that a high occupancy of the CPU is caused. In addition, the CPU has to control data input/output at the DSP and DSC. Thus, an excessive load may be applied to the CPU in forming the ultrasound image.

### BRIEF DESCRIPTION OF THE DRAWING

FIG. 1 is a block diagram showing an ultrasound imaging system in accordance with a first embodiment of the present invention.

FIG. 2 is a schematic diagram showing an example of vertex initialization on a screen displaying an ultrasound image.

FIG. 3 is a schematic diagram showing an example of forming texture data.

FIG. 4 is a block diagram showing an ultrasound imaging system in accordance with a second embodiment of the present invention.

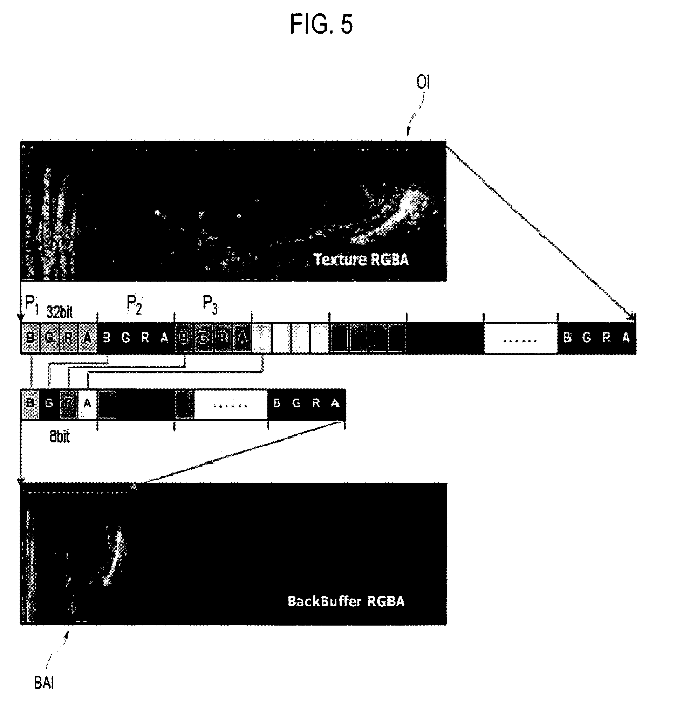

FIG. 5 is a schematic diagram showing an example of byte alignment conversion.

FIG. 6 is a block diagram showing an ultrasound imaging system in accordance with a third embodiment of the present invention.

FIG. 7 is a block diagram showing an ultrasound imaging system in accordance with a fourth embodiment of the present invention.

FIG. 8 is a block diagram showing an ultrasound imaging system in accordance with a fifth embodiment of the present invention.

FIG. 9 is a block diagram showing an ultrasound imaging system in accordance with a sixth embodiment of the present invention.

### DETAILED DESCRIPTION OF THE INVENTION

First Embodiment

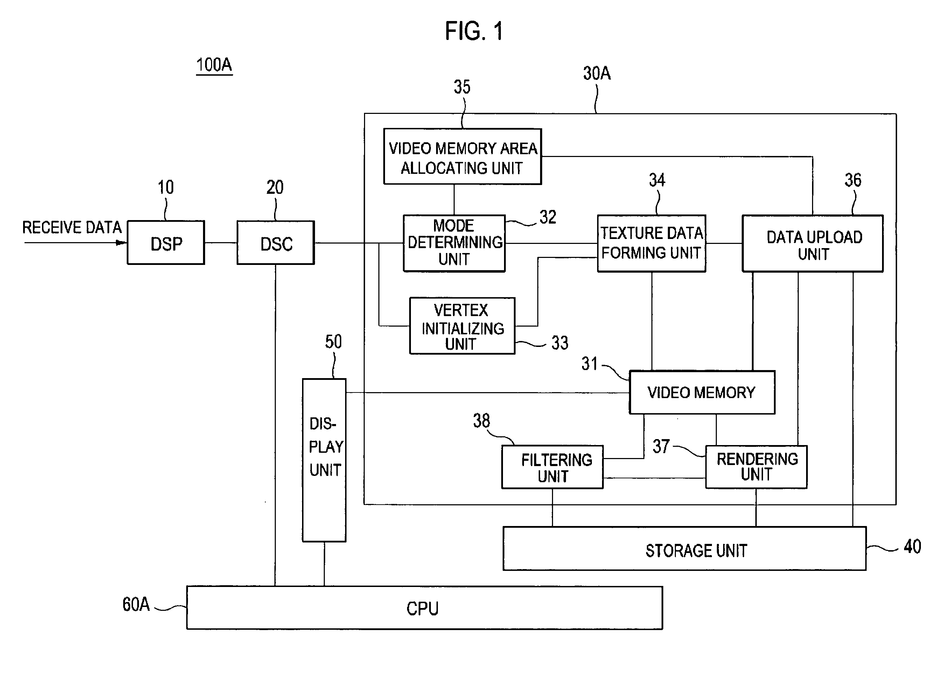

FIG. 1 is a block diagram showing an ultrasound imaging system in accordance with a first embodiment of the present invention. The ultrasound imaging system 100A may include a digital signal processor (DSP) 10, a digital scan converter (DSC) 20 and a graphic processing unit (GPU) 30A. The DSP may be operable to process 8-bit receive data outputted from an ultrasound diagnostic unit (not denoted) to thereby form 8-bit raw data for forming a brightness (B) mode image, a color (C) mode image or a Doppler (D) mode image. The DSC 20 may be operable to scan convert the 8-bit raw data to output scan-converted data having a data format suitable for display. The GPU 30A may be operable to perform rendering and filtering upon the scan-converted data to form pixel data.
Detailed explanations of functions of the GPU 30A will be described later.

A storage unit 40 may store color palette data, a shader code for application of the color palette data, a filter shader code for filtering, a color keying code for transparency treatment and a color information mapping code. A display unit 50 may include a monitor and display an ultrasound image based on pixel data provided from the GPU 30A. The CPU 60A may be operable to control the operations of the DSP 10, DSC 20, GPU 30A, storage unit 40 and display unit 50.

A video memory 31 in the GPU 30A may include a plurality of storage areas including a storage area for the pixel data corresponding to pixels consisting of a screen of the display unit 50. That is, the pixel data formed in GPU 30A are stored at the corresponding storage area of the video memory and displayed on a screen of the display unit 50 at the same time. Thus, the necessary capacity of the video memory 31 may depend on the number of pixels and data format for each of the image modes. For example, in case of an image of 640*480 pixels, a minimum capacity of the video memory may be 640*480*8 bits. The data format may be different according to the image modes. For example, the B-mode image and a loop mode image may have an 8-bit data format. Also, the C-mode image and a marker may have a 16-bit data format. The marker may represent a display range on the screen of the display unit. A mode determination 32 may be operable to determine an image mode from the scan-converted data inputted to the GPU 30A to thereby form mode information.

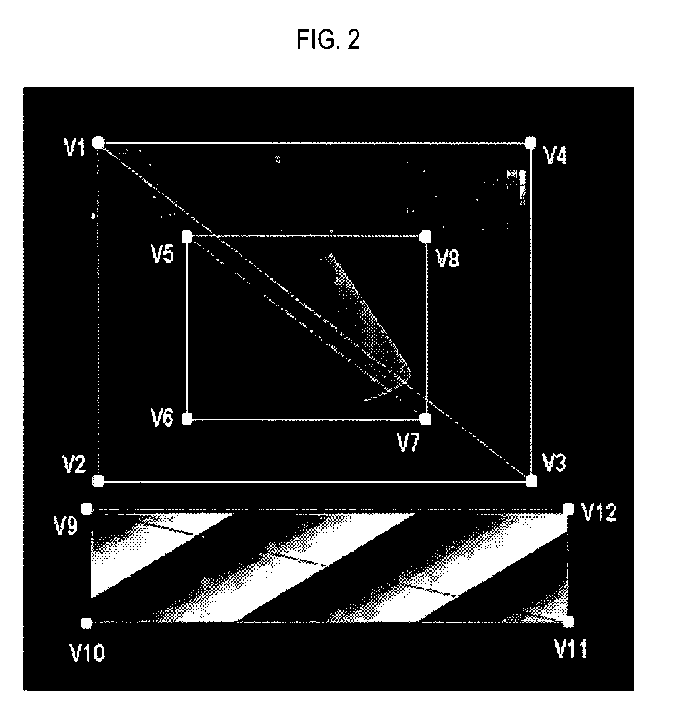

If output data of the DSC 20, i.e., the scan-converted data, are inputted, then a vertex initializing unit 33 in the GPU 30A may be operable to form vertex information for initializing a position for displaying an image on the screen of the display unit 50 based on vertexes. The vertex initializing unit 33 may be embodied with a 3-dimensional application programming interface (3D APT). Referring to FIG. 2, when a marker, a B-mode image and a loop mode image are formed, the vertex initializing unit 33 may be operable to initialize positions of vertexes V1, V2, V3 and V4 of the marker and vertexes V5, V6, V7 and V8 of a B-mode image as well as vertexes V9, V10, V11 and V12 of the loop image. For example, a first polygon defined by vertexes V1, V2 and V3, a second polygon defined by vertexes V3, V4 and V1, a third polygon defined by vertexes V5, V6 and V7, a fourth polygon defined by V7, V8 and V5, a fifth polygon defined by vertexes V9, V10 and V11, and a sixth polygon defined by V11, V12 and V9 may be formed. Herein, the number of the vertexes or polygons is merely an example. The polygons may be formed by using 30-40 vertexes.

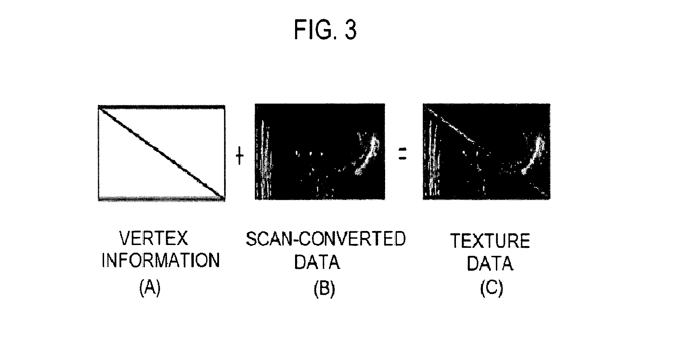

Textures of a 3D graphic format should be used to upload an ultrasound image, a marker image and the like to the video memory. A texture data forming unit 34 may be operable to combine vertex information A formed at the vertex initializing unit 33 with the scan-converted data B based on the mode information outputted from the mode determining unit 32 to thereby form texture data C, as shown in FIG. 3. For example, when the B-mode image or the loop-mode image having an 8-bit data format is formed, texture data having a "D3DFMT_L8" format, which is an 8-bit only format, are formed. Also, when the C-mode image of a 16-bit data format is formed, texture data of a "D3DFMT_R5G6B5" format, which is a 16-bit only format, are formed.

A video memory area allocating unit 35 may be operable to analyze the mode information outputted from the mode determining unit 32 and form storage area allocation information of the video memory 31 based on a data format corresponding to the analyzed mode. The storage area allocation information may include storage area information of the texture data and storage area information of various codes for performing the functions of the GPU. Storage area information of the color palette data may be further included according to a desirable image mode. For example, if the analyzed mode represents the C mode of a 16-bit data format, then the video memory area allocating unit 35 may form storage area allocation information about a storage area for 16-bit scan-converted data of pixels, a storage area for frame data obtained by rendering the scan-converted data, a storage area for pixel data obtained by filtering the frame data, a storage area for a shader code and a storage area for color palette data.

The data upload unit 36 may be operable to upload the texture data, the shader code or the palette information to the storage areas allocated in the video memory 31 based on the storage area allocation information. When the C-mode image is formed as mentioned above, the data upload unit 36 may be operable to upload the scan-converted data outputted from the DSC 20, the color palette data stored in the storage unit 40 and the shader code to the storage areas allocated in the video memory 31. When the upload is completed, the data upload unit 36 may be operable to form a data upload completion signal.

A rendering unit 37 may be operable to perform rendering upon the texture data based on the shader code uploaded into the video memory 31 in response to the data upload completion signal to thereby form the frame data. For example, the scan-converted data may be rendered by using the color palette based on the shader code. The frame data may have a 32-bit data format. The frame data formed at the rendering unit of the GPU 30A may be stored at a storage area allocated in the video memory 31. If the rendering is completed, then the rendering unit 37 may be operable to generate a rendering completion signal.

In response to the rendering completion signal, the filtering unit 38 may be operable to perform filtering upon the rendered data, which applies the filter shader code provided from the storage unit 40 to the frame data to thereby form the pixel data. The pixel data may be stored in a storage area allocated in the video memory 31. As the frame data obtained by the rendering are stored in the video memory 31 in accordance with the first embodiment of the present invention, the rendering results may be used by other modules such as the filtering unit.

The CPU 60A of the ultrasound imaging system may be merely operable to control data input/output of the DSP 10, DSC 20, GPU 30A and display unit 50. This is so that the load of the CPU 60A may be considerably decreased.

The functions of the mode determining unit 32, the vertex initializing unit 33, the texture data forming unit 34, the video memory area allocating unit 35, the rendering unit 37 and the filtering unit 38 included in the GPU 30A mentioned in the first embodiment of the present invention may be embodied by Direct3D.

Second Embodiment

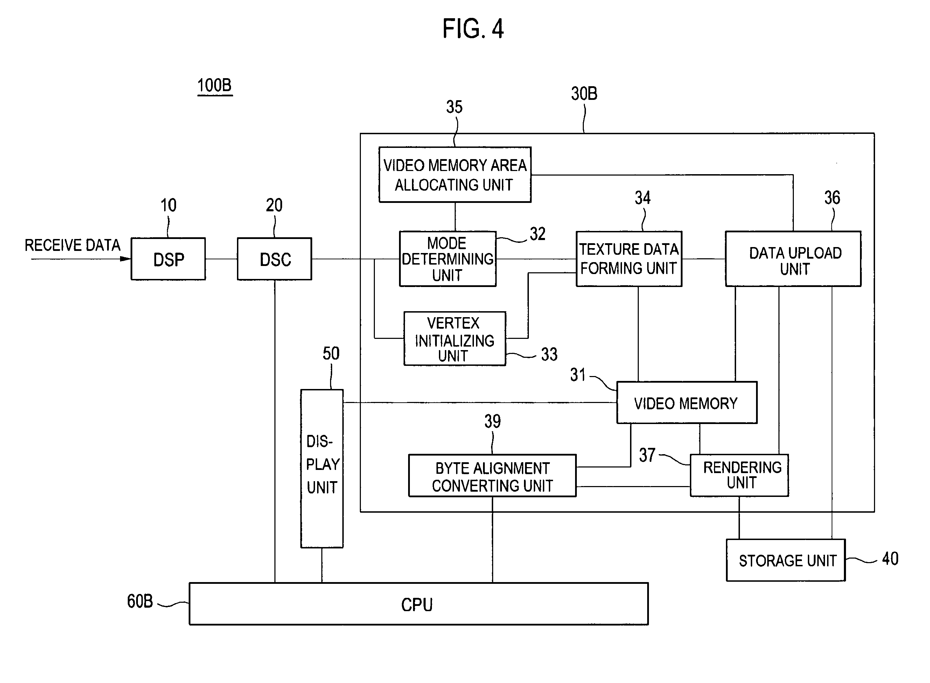

FIG. 4 is a block diagram showing an ultrasound imaging system in accordance with a second embodiment of the present invention. Filtering in the ultrasound imaging system 100B may be carried at a CPU 60B. The CPU 60B may be operable to process 8-bit or 16-bit data. A GPU 30B of the second embodiment may include a byte alignment converting unit 39 instead of the fingering unit 38 in the GPU 30A of the first embodiment. The byte alignment converting unit 39 may be operable to convert 32-bit frame data to 8-bit or 16-bit frame data according to the image mode by using the shader code for byte alignment. For example, pixels P1, P2, ... Pn of an original texture image OA, which are indicated by frame data formed at the rendering unit 37, may be expressed as 32-bit data including four 8-bit sub pixel data "R," "G," "B" and "A." "R," "G" and "B" may represent color information of pixels, i.e., red, green and blue. "A" may represent an alpha value indicating transparency. In the conventional imaging processing device, "R," "G," "B" and "A" may have values of 0 to 255. The CPU may convert the values of "R," "G," "B" and "A" to values of 0.0 to 1.0 for use therein. The byte alignment converting unit 39 may be operable to extract one of the sub pixel date "R," "G," "B" and "A" from each of the pixel data. In such a case, different types of the sub pixel data are extracted from neighboring pixel data. Accordingly, a byte alignment image BAI, whose pixels P1, P2 ... Pn are indicated with the extracted 8-bit sub pixel data, may be obtained.

Elements and their functions, which are not described among the elements of the ultrasound imaging system 100B shown in FIG. 4, may be identical or similar to those of the ultrasound imaging system 100A shown in FIG. 1. Thus, detailed descriptions thereof will be omitted herein.

Third Embodiment

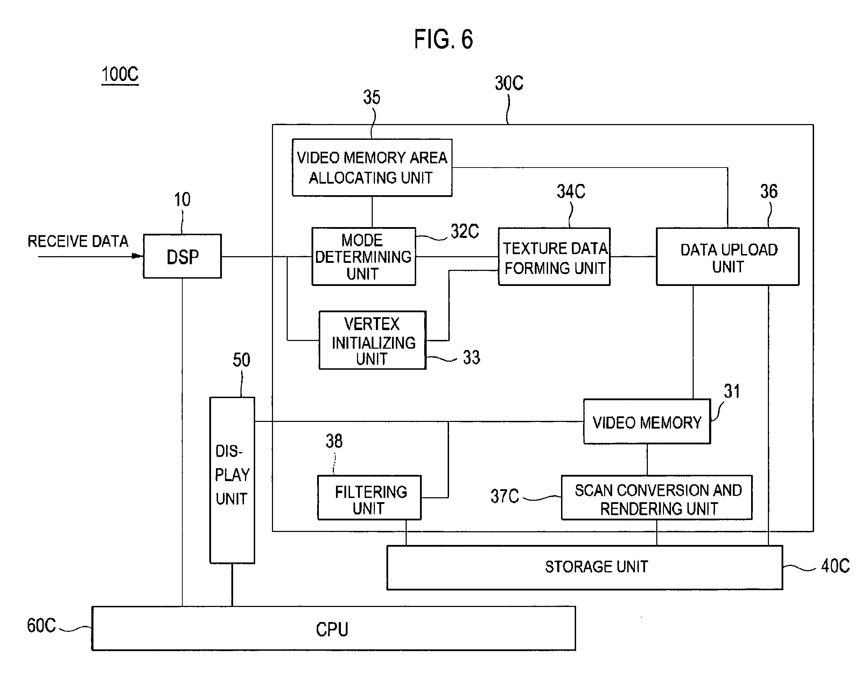

FIG. 6 is a block diagram showing an ultrasound imaging system in accordance with a third embodiment of the present invention. Referring to FIG. 6, the GPU 30C of the third embodiment may be operable to perform scan conversion in addition to all of the functions of the GPU 30A of the first embodiment mentioned above. For this, a scan conversion shader code may be additionally stored in the storage unit 40C. The scan conversion shader code may a code complied with a binary file for security. The scan conversion may be carried out by using a lookup table in real time or by calculating scan conversion data for all of the data. The scan conversion unit 20 shown in the first embodiment may be omitted in the third embodiment.

The mode determining unit 32C of the GPU 30C may be operable to determine an image mode from 8-bit receive data formed at the DSP 10 to thereby form mode information in accordance with the third embodiment of the present invention. The vertex initializing unit 33 may be operable to initialize vertexes according to the size of an image. If the initializing is completed, then the texture data forming unit 34C may combine the vertex information formed at the vertex initializing unit 33 with the image data based on the mode information outputted from the mode determining unit 32C to thereby form texture data.

Similar to the first embodiment, the data upload unit 36 may upload the texture data, various shader codes including the scan conversion shader code or palette information to the video memory 31.

The scan conversion and rendering unit 37C may perform scan conversion and rendering upon the texture data based on the shader code uploaded at the video memory 31 in response to the data upload completion signal to thereby form the frame data. The frame data formed at the scan conversion and rendering unit 37C may be stored at a storage area allocated in the video memory 31. If the scan conversion and the rendering are completed, then a rendering completion signal is generated.

Elements and their functions, which are not described among the elements shown in FIG. 6 such as the filtering unit 38, CPU 60C, etc., may be identical or similar to those of the ultrasound imaging system 100A shown in FIG. 1. Thus, detailed description thereof will be omitted herein.

Fourth embodiment

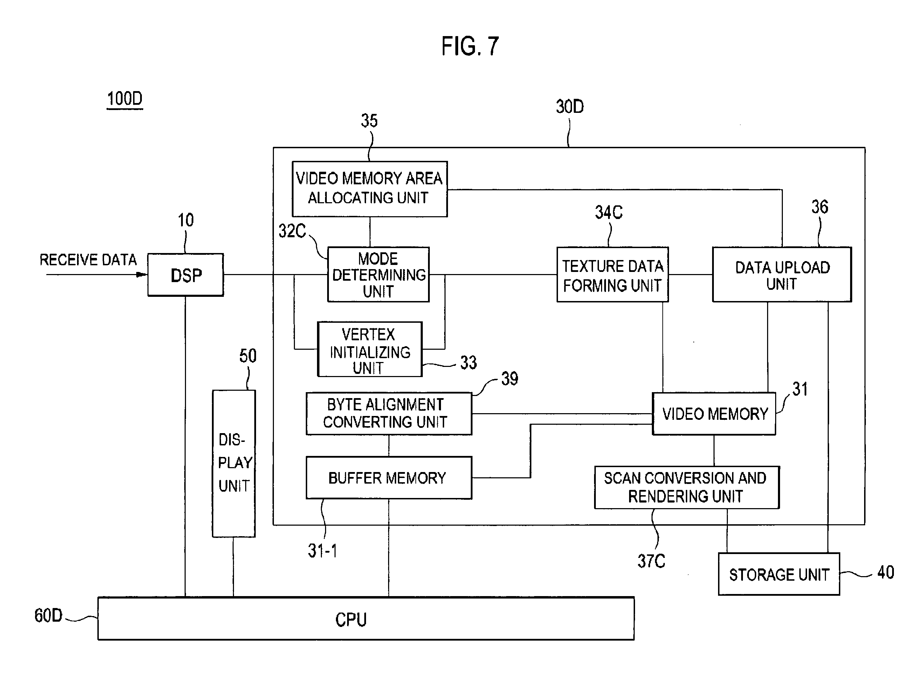

FIG. 7 is a block diagram showing an ultrasound imaging system in accordance with fourth embodiment of the present invention. An image processing unit of the ultrasound imaging system 100D may include a DSP 10 and a GPU 30D. Scan conversion and filtering may be carried out at the GPU 30D. The CPU 60D may process 8-bit or 16-bit data in the same manner as the second embodiment. The GPU 30D may include a byte alignment converting unit 39 instead of the filter unit 38 shown in the third embodiment. The byte alignment converting unit 39 may be operable to convert 32-bit frame data to 8-bit or 16-bit frame data according to the mode by using the shader code for byte alignment.

The frame data formed by the byte alignment converting unit 39 may be stored in a buffer memory 31_1. The buffer memory 31_1 may be a system memory. The frame data stored in the buffer memory 31_1 may be used at CPU based image processing modules.

Elements and their functions, which are not described among the elements shown in FIG. 7, may be identical or similar to those of the third embodiment shown in FIG. 4. Thus, detailed descriptions thereof will be omitted herein.

Fifth embodiment

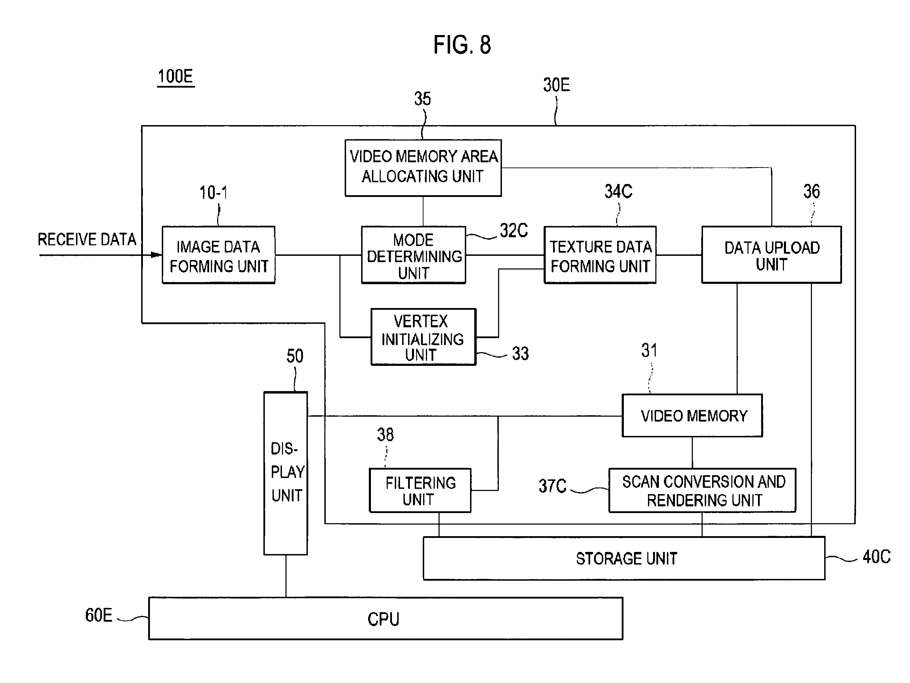

FIG. 8 is a block diagram showing an ultrasound imaging system in accordance with a fifth embodiment of the present invention. An image processing unit of the ultrasound imaging system 100E may include a GPU 30E. Referring to FIG. 8, the GPU 30E may be operable to receive the receive data and form image data based on the receive data, in addition to all of the functions of the GPU 30C shown in FIG. 6 of the third embodiment. For this, the GPU 30E may further include an image data forming unit 10_1. The image data forming unit 10_1 may be operable to process the 8-bit receive data outputted from the ultrasound diagnostic unit (not shown) to thereby form B-mode image data, C-mode image data, D-mode data or the like. In the fifth embodiment, the DSP 10 shown in FIG. 6 of the third embodiment may be omitted.

Elements and their functions, which are not described among the elements shown in FIG. 8, may be identical or similar to those of the first or third embodiment. Thus, detailed descriptions thereof will be omitted herein.

Sixth Embodiment

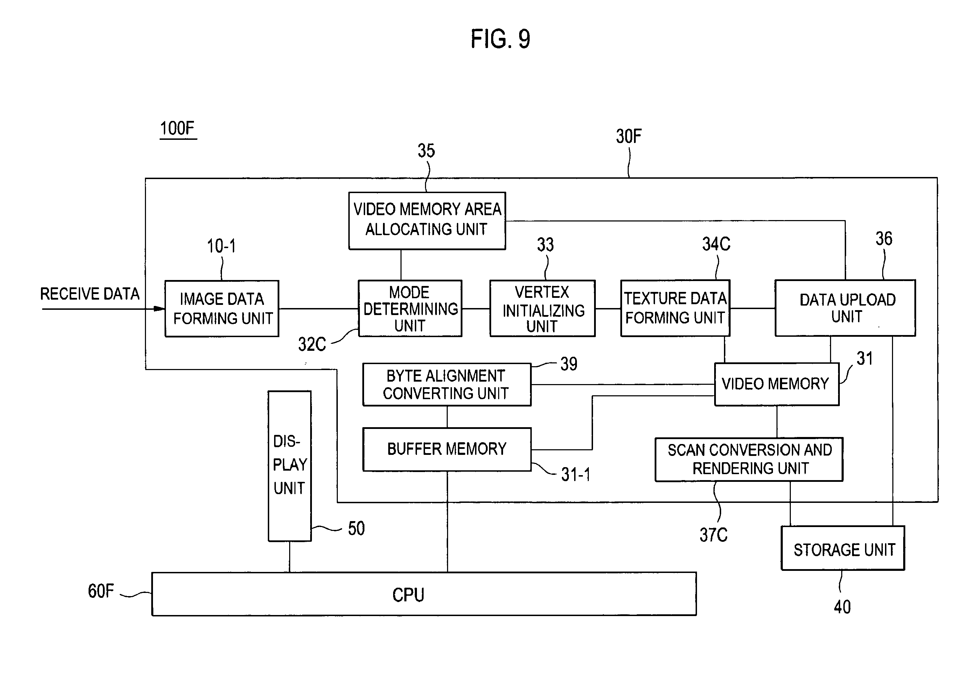

FIG. 9 is a block diagram showing an ultrasound imaging system 100F in accordance with a sixth embodiment of the present invention. A GPU 30F may include a byte alignment converting unit 39 instead of the filtering unit 38 shown in the fifth embodiment of the present invention. The byte alignment converting unit 39 may be operable to form the frame data. The frame data may be stored in the buffer memory 31_1. The buffer memory 31_1 may be a system memory. The frame data stored in the buffer memory 31_1 may be used in CPU based image processing modules.

Elements and their functions, which are not described among the elements of the ultrasound imaging system 100F shown in FIG. 9, may be identical or similar to those of the fourth embodiment. Thus, detailed descriptions thereof will be omitted herein.

In accordance with one embodiment of the present invention, there is provided an ultrasound imaging system, comprising: an ultrasound diagnostic unit operable to transmit ultrasound signals to a target object and form receive data based on ultrasound echo signals reflected from the target object; and an image processing unit operable to form an ultrasound image based on the receive data, the image processing unit including a graphic processing unit configured to perform at least one of functions including processing the receive data to form image data, performing scan conversion upon the image data to form scan-converted data suitable for display, and rendering and filtering the scan-converted data to form pixel data.

In accordance with another embodiment of the present invention, there is provided an imaging device, comprising: a touch screen operable to display a plurality of soft buttons for allowing a user to input an instruction, said instruction including a request for designating one of the soft buttons and changing attributes of the designated soft button, said each of the attributes having an attribute name and a plurality of attribute values and said instruction further including a change information for changing an attribute value of the designated soft button; a control unit operable to change the attribute value of the designated soft button based on the attribute change information; and a storing unit operable to store attributes of the soft buttons.

Any reference in this specification to "one embodiment," "an embodiment," "example embodiment," etc. means that a particular feature, structure or characteristic described in connection with the embodiment is included in at least one embodiment of the present invention. The appearances of such phrases in various places in the specification are not necessarily all referring to the same embodiment. Further, when a particular feature, structure or characteristic is described in connection with any embodiment, it is submitted that it is within the purview of one skilled in the art to effect such feature, structure or characteristic in connection with other ones of the embodiments.

Although embodiments have been described with reference to a number of illustrative embodiments thereof, it should be understood that numerous other modifications and embodiments can be devised by those skilled in the art that will fall within the spirit and scope of the principles of this disclosure. More particularly, numerous variations and modifications are possible in the component parts and/or arrangements of the subject combination arrangement within the scope of the disclosure, the drawings and the appended claims. In addition to variations and modifications in the component parts and/or arrangements, alternative uses will also be apparent to those skilled in the art.

## Claims

1. An ultrasound imaging system, comprising:
an ultrasound diagnostic unit operable to transmit ultrasound signals to a target object and form receive data based on ultrasound echo signals reflected from the target object; and
an image processing unit operable to form an ultrasound image based on the receive data, the image processing unit including a graphic processing unit configured to perform at least one of functions including processing the receive data to form image data, performing scan conversion upon the image data to form scan-converted data suitable for display, and rendering and filtering the scan-converted data to form pixel data.

2. The ultrasound imaging system of Claim 1, further comprising a storage unit configured to store at least one of a shader code for application of a color palette, a color keying code for transparency treatment and a color information mapping code.

3. The ultrasound imaging system of Claim 2, further comprising a display unit operable to display the ultrasound image

4. The ultrasound imaging system of Claim 3, wherein the image processing unit further includes a central processing unit operable to control at least one of the storage unit and the display unit.

5. The ultrasound imaging system of Claim 4, wherein the graphic processing unit includes:
a video memory having a plurality of storage areas;
a mode determining unit operable to determine an image mode based on the scan-converted data to form mode information;
a vertex initializing unit operable to form vertex information based on the scan-converted data;
a texture data forming unit operable to combine the vertex information with the scan-converted data to thereby form texture data;
a video memory area allocating unit operable to form storage area allocation information of the video memory according to a data format of the image mode based on the mode information;
a data upload unit operable to upload the texture data to a storage area allocated at the video memory based on the storage area allocation information;
a rendering unit operable to perform rendering upon the texture data uploaded at the video memory to form frame data; and
a filtering unit operable to perform filtering upon the frame data to form pixel data.

6. The ultrasound imaging system of Claim 4, wherein the image processing unit includes:
a digital signal processing unit operable to process the receive data to form the image data; and
a digital scan converting unit operable to perform scan conversion upon the image data to form digital scan-converted data,
wherein the graphic processing unit includes:
a video memory having a plurality of storage areas;
a mode determining unit operable to determine an image mode based on the digital scan-converted data to form mode information;
a vertex initializing unit operable to form vertex information based on the digital scan-converted data;
a texture data forming unit operable to combine the vertex information with the scan-converted data to thereby form texture data;
a video memory area allocating unit operable to form storage area allocation information of the video memory according to a data format of the image mode based on the mode information;
a data upload unit operable to upload the texture data to a storage area allocated at the video memory based on the storage area allocation information;
a rendering unit operable to perform rendering upon the texture data uploaded at the video memory to form frame data; and
a byte alignment converting unit operable to convert a data format of the frame data,
wherein the CPU is configured to perform filtering upon the frame data to form the pixel data.

7. The ultrasound imaging system of Claim 4, wherein the image processing unit includes a digital signal processing unit operable to process the receive data to form the image data, and wherein the graphic processing unit is operable to scan-convert the image data and render and filter the scan-converted image data to thereby form the pixel data.

8. The ultrasound imaging system of Claim 7, wherein the graphic processing unit includes:
a video memory having a plurality of storage areas;
a mode determining unit operable to determine an image mode based on the scan-converted data to form mode information;
a vertex initializing unit operable to form vertex information based on the scan-converted data;
a texture data forming unit operable to combine the vertex information with the scan-converted data to thereby form texture data;
a video memory area allocating unit operable to form storage area allocation information of the video memory according to a data format of the image mode based on the mode information;
a data upload unit operable to upload the texture data to a storage area allocated at the video memory based on the storage area allocation information;
a rendering unit operable to perform rendering upon the texture data uploaded at the video memory to form frame data; and
a filtering unit operable to perform filtering upon the frame data to form pixel data.

9. The ultrasound imaging system of Claim 3, wherein the image processing unit includes a digital signal processing unit,
wherein the graphic processing unit includes:
a video memory having a plurality of storage areas;
a mode determining unit operable to determine an image mode based on the digital scan-converted data to form mode information;
a vertex initializing unit operable to form vertex information based on the digital scan-converted data;
a texture data forming unit operable to combine the vertex information with the scan-converted data to thereby form texture data;
a video memory area allocating unit operable to form storage area allocation information of the video memory according to a data format of the image mode based on the mode information;
a data upload unit operable to upload the texture data to a storage area allocated at the video memory based on the storage area allocation information;
a rendering unit operable to perform rendering upon the texture data uploaded at the video memory to form frame data; and
a byte alignment converting unit operable to convert a data format of the frame data,
wherein the CPU is configured to perform filtering upon the frame data to form the pixel data.

10. The ultrasound imaging system of Claim 3, wherein the graphic processing unit includes:
an image data forming unit operable to process the receive data to form image data;
a video memory having a plurality of storage areas;
a mode determining unit operable to determine an image mode based on the scan-converted data to form mode information;
a vertex initializing unit operable to form vertex information based on the scan-converted data;
a texture data forming unit operable to combine the vertex information with the scan-converted data to thereby form texture data;
a video memory area allocating unit operable to form storage area allocation information of the video memory according to a data format of the image mode based on the mode information;
a data upload unit operable to upload the texture data to a storage area allocated at the video memory based on the storage area allocation information;
a rendering unit operable to perform rendering upon the texture data uploaded at the video memory to form frame data; and
a filtering unit operable to perform filtering upon the frame data to form pixel data.

11. The ultrasound imaging system of Claim 3, wherein the graphic processing unit includes:
a video memory having a plurality of storage areas;
a mode determining unit operable to determine an image mode based on the digital scan-converted data to form mode information;
a vertex initializing unit operable to form vertex information based on the digital scan-converted data;
a texture data forming unit operable to combine the vertex information with the scan-converted data to thereby form texture data;
a video memory area allocating unit operable to form storage area allocation information of the video memory according to a data format of the image mode based on the mode information;
a data upload unit operable to upload the texture data to a storage area allocated at the video memory based on the storage area allocation information;
a rendering unit operable to perform rendering upon the texture data uploaded at the video memory to form frame data; and
a byte alignment converting unit operable to convert a data format of the frame data,
wherein the CPU is configured to perform filtering upon the frame data to form the pixel data.

12. The ultrasound imaging system of Claim 11, wherein the graphic processing unit further includes a buffer memory for storing the byte alignment converted data.
